Europäisches Patentamt

(19) European Patent Office

Office européen des brevets

(11) Publication number: **0 136 103**
**B1**

(12) **EUROPEAN PATENT SPECIFICATION**

(45) Date of publication of patent specification: **31.10.90**

(21) Application number: **84305911.4**

(22) Date of filing: **29.08.84**

(51) Int. Cl.⁵: **A 61 K 31/18,** A 61 K 9/22,
A 61 K 47/00

(54) Amosulalol hydrochloride long acting formulations.

(30) Priority: **31.08.83 JP 160086/83**

(43) Date of publication of application:
**03.04.85 Bulletin 85/14**

(45) Publication of the grant of the patent:
**31.10.90 Bulletin 90/44**

(84) Designated Contracting States:
**AT BE CH DE FR GB IT LI NL SE**

(56) References cited:
**DE-A-2 645 547**
**DE-A-2 843 016**
**US-A-3 784 683**

**Hagers Handbuch der pharmazeutischen Praxis,
1977, Band VII, Teil B, Seiten 400-403**

The file contains technical information
submitted after the application was filed and
not included in this specification

(73) Proprietor: **YAMANOUCHI PHARMACEUTICAL
CO. LTD.**
**No. 3-11 Nihonbashi-Honcho, 2-chome Chuo-ku
Tokyo (JP)**

(72) Inventor: **Sonobe, Takashi**
**No. 4-10-12, Fujimi Fukiage-cho
Kitaadachi-gun Saitama (JP)**
Inventor: **Sugiura, Hiroshi**
**No. 6-8-3, Arajuka-cho
Kawagoe-shi Saitama (JP)**
Inventor: **Itoh, Tomoh**
**No. 3-16-1, Hasune-cho Itabashi-ku
Tokyo (JP)**
Inventor: **Aruga, Masayoshi**
**No. 5-16-14, Midorigaoka
Ageo-shi Saitama (JP)**
Inventor: **Kawata, Hiroitsu**
**No. 1-7, Oaza Namiki
Kawagoe-shi Saitama (JP)**

(74) Representative: **Geering, Keith Edwin et al
REDDIE & GROSE 16 Theobalds Road
London WC1X 8PL (GB)**

Courier Press, Leamington Spa, England.

## Description

This invention relates to a long acting formulation of amosulalol hydrochloride (chemical name: 5-{1-hydroxy-2-[2-(2-methoxyphenoxy)-ethyl-amino]ethyl}-2-methylbenzenesulfonamide hydrochloride).

Amosulalol hydrochloride is an excellent hypotensive agent having an adrenergic α-blocking action and an adrenergic β-blocking action. In general, it is desirable that the dosing frequency of a hypotensive agent be low, for simplicity of clinical therapy, but the biological half life of amosulalol hydrochloride has made this difficult to achieve.

It is known from DE—A—2843016 that compounds such as amosulalol and addition salts thereof with pharmaceutically acceptable acids can be prepared in formulations containing conventional additives and excipients. An exmaple illustrates granulating active component with excipients using a starch paste binder and then molding the resulting formulation into tablets. It is proposed to administer orally 50-300 mg of active compound three times per day.

It is known from US Patent 3784683 to make tablets containing as active ingredient a drug specifically designed to act in the large intestine in order to combat bacteria and parasites. The dosage form for oral administration is prepared by coating or embedding finely divided particles of the chosen drug in a relatively insoluble pharmaceutically acceptable resin. The resin completely covers the particles of drug and the drug is not exposed to the intestinal fluids. Eventually in the large intestine the coating is worn down to expose the drug, which can then start to take its effect. It is stated that patients who ingest such a tablet will show substantially no blood-level of the drug, yet suitable analysis of faeces will show that substantially all of the drug is dissolved and available for topical appliction to the large intestine.

It is known from DE—A—2645547 to make a controlled release tablet containing a medicinal agent (eg a sulphonamide) in a core material formed from two specially selected polymeric materials which form a gummy water insoluble complex of gel-like consistency under the action of water or gastric fluid, and to coat the core in a substantially water-insoluble water-permeable rupturable film coating formed of blended hydrophobic and hydrophilic polymers. The gel retards the dissolution of the drug out of the tablet and, while the film is intact, the release of the drug is primarily controlled by diffusion of solvent and solute molecules through the film. Swelling of the gummy complex causes the film to rupture or erode and release rate is then controlled by the gummy complex. Examples are given of the tablet-like water insoluble matrix in which the active ingredient is dispersed, the tablet being coated with a water-soluble film of low water solubility. The release rate of active drug from uncoated cores and the claimed film coated tablets was compared, the combination of core matrix and film being considered essential to the claimed invention.

Coating agents for tablets, and binding agents for tablets, of various descriptions are known in the art. There are known types of composition which are respectively primarily entero-soluble, primarily gastro-soluble, entero-insoluble or gastro-insoluble. Some compositions are primarily insoluble over the whole range of conditions of the gastro-intestinal tract but are able to absorb water so as to swell over a period of time; the phenomenon of swelling of a tablet binding agent may be ulilised to effect a delayed drug release. Some examples of the types of compositions referred to above are to be found in "Hagers Handbuch Der Pharmazeutischen Praxis, 1977, Volume VII, Part B, 400—403" where a range of commercially available "Eudragit" (TM) acrylic acid/acrylate copolymer compounding agents are discussed.

As the result of various investigations, the inventors have discovered that a formulation prepared from amosulalol hydrochloride and between 10% and 30% by weight of entero-soluble material in intimate admixture can have long acting characteristics; and further that by including pharmaceutically acceptable organic acid in the formulation, the solubilization of amosulalol hydrochloride in a high pH range can be promoted and the bio-availability thereof increased.

Accordingly this invention provides a long acting formulation comprising amosulalol hydrochloride and between 10% and 30% w/w entero-soluble material in intimate admixture, and optionally pharmaceutically acceptable organic acid.

By the formulation of this invention, it becomes possible to maintain the minimum effective plasma level of amosulalol hydrochloride for a long period without initially increasing the plasma level unduly beyond what is necessary.

In the accompanying drawings:

Fig. 1 shows the plasma level of amosulalol hydrochloride in a single-blind cross-over study as a function of time following oral administration for a) the long acting formulation of this invention prepared in Example 1 and b) a conventional formulation of amosulalol hydrochloride;

Fig. 2 is a corresponding graph for the long acting formulation of this invention prepared in Example 2 and for a conventional formulation of amosulalol hydrochloride (mean of five determinations in beagle dogs); and

Fig. 3 is a corresponding graph for the long acting formulation of this invention prepared in Example 3 and for an aqueous solution of amosulalol hydrochloride (mean of six determinations in beagle dogs).

A formulation of this invention can be example be prepared in the following manner:

Amosulalol hydrochloride is mixed well with excipient (which may be conventional) and after adding thereto a solution or a suspension of

entero-soluble material in water or in an organic solvent, the resultant mixture is granulated. In this case the entero-soluble material may be directly added to the aforesaid mixture of amosulalol and, after adding thereto a binder (which may be conventional), the resultant mixture may be granulated.

Pharmaceutically acceptable organic acid may be added to the aforesaid mixture before granulation. Furthermore, the granules thus obtained may be formed into tablets by means of a tableting machine and coating may be applied to these tablets to prevent bitterness and improve appearance.

Examples of the entero-soluble material useable in this invention are a methacrylic acid/ethyl acrylate copolymer (e.g., Eudragit L30D-55, trade name made by Rohm and Haas Company, a copolymer of methacrylic acid and ethyl acrylate (1:1) having a molecular weight of about 250,000); a methacrylic acid/methyl methacrylate copolymer (e.g., Eudragit L100, trade name, made by Rohm and Haas Company, a copolymer of methacrylic acid and methyl methacrylate (1:1) having a molecular weight of 135,000 or Eudragit® S100, a copolymer of methacrylic acid and methyl methacrylate (1:2) having a molecular weight of about 135,000); hydroxypropylmethyl cellulose phthalate (The Japan Pnarmacopoeia, 10th Revision); cellulose acetate phthalate (The Japan Pharmacopoeia, 10th Revision); shellac (The Japan Pharmacopoeia, 10th Revision), etc.

These entero-soluble materials are soluble at different minimum pH values. For example, Eudragit® L30D-55 is soluble at a pH higher than about 5.5,

Eudragit® L100 at a pH higher than about 6.0, and

Eudragit® S100 at a pH higher than about 7.0.

The selection of entero-soluble material used can determine the intestinal portion(s) at which the medicament is absorbed, and its long acting characteristics can thus be controlled. Among the above-described entero-soluble materials, methacrylic acid/ethyl acrylate copolymers can be dissolved in water; this case is safe and economical, and the granulation is easier than when using an organic solvent.

In addition, a methacrylic acid/ethyl acrylate copolymer (Eudragit® L30D-55) is commercially available in the form of an aqueous (usually 30%) dispersion.

The entero-soluble material constitutes 10 to 30% by weight of the total weight of the formulation of this invention.

Examples of the pharmaceutically acceptable organic acid optionally used in this invention are citric acid (The Japanese Pharmacopoeia, 10th Revision), tartaric acid (The Japan Pharmacopoeia, 10th Revision), etc. The purpose of using the organic acid is to improve the solubility of amosulalol hydrochloride at a high pH region (in particular, about 7.5 which is one of the pH values of a physiological saline solution), whereby the bio-availability of amosulalol hydrochloride is increased. The content of the organic acid is e.g. 1 to 30% of the total weight of the product, preferably 5 to 20% by weight.

In this invention, excipients, lubricants, binders, etc., which are used for conventional formulations can be used without particular restrictions. Examples of the excipients are lactose, starch, calcium hydrogenphosphate, silicic anhydride, etc. Examples of the lubricants are magnesium stearate, talc, etc.; and examples of the binders are hydroxypropyl cellulose, starch, etc. There is no particular restriction on the amounts of these additives; these amounts may be selected according to the intended end use.

The present invention is illustrated by the following Examples.

Example 1

In a fluidized bed granulator 500 g of amosulalol hydrochloride and 500 g of lactose were mixed. Into the mixture was sprayed an aqueous dispersion of a methacrylic acid/ethyl acrylate copolymer (Eudragit® L30D-55) in an amount of 240 g (solid component) and granules were formed from the mixture by use of the fluidized bed granulator. After drying the granules thus obtained for 4 hours at 40°C, 6 g of magnesium stearate was added to the granules and the mixture was formed into tablets by means of an ordinary tableting machine.

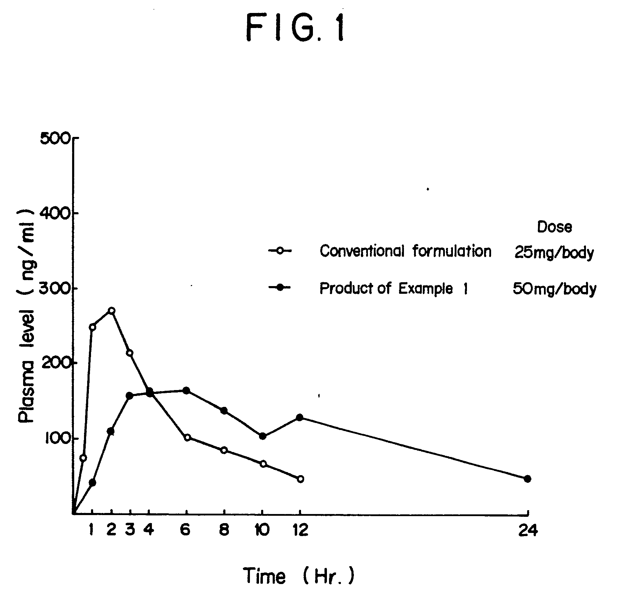

The tablets of this invention (containing 50 mg of amosulalol hydrochloride) and of conventional formulation (containing 25 mg of amosulalol hydrochloride and no entero-soluble material) were orally administered once a day to healthy adult men by a crossover method with a wash-out period of one week. In the case of using the tablets of this invention, the amosulalol hydrochloride concentration in the plasma was measured by high pressure liquid chromatographic analysis at 1, 2, 3, 4, 6, 8, 10, 12 and 24 hours after the administration, while for the conventional tablets, the amosulalol hydrochloride concentration in the plasma was measured in the same manner at 1, 2, 3, 4, 6, 8, 10, and 12 hours after the administration. The results are shown in Fig. 1.

As shown in the Figure, it can be seen that by administration of the formulation of this invention daily, provision of a suitable level of amosulalol hydrochloride in the plasma can be prolonged as compared to the case of using the conventional formulation.

Example 2

In a fluidized bed dryer were mixed 200 g of amosulalol hydrochloride, 50 g of silicic anhydride and 30 g of hydroxypropyl cellulose and after spraying thereon a solution prepared by dissolving 20 g of citric acid in an aqueous dispersion of a methacrylic acid/ethyl acrylate copolymer (Eudragit® L30D-55), granules were produced from the mixture using a fluidized bed granulator. To the granules thus formed was added 1.6 g of magnesium stearate and the mixture was formed into tablets by means of an ordinary tableting machine.

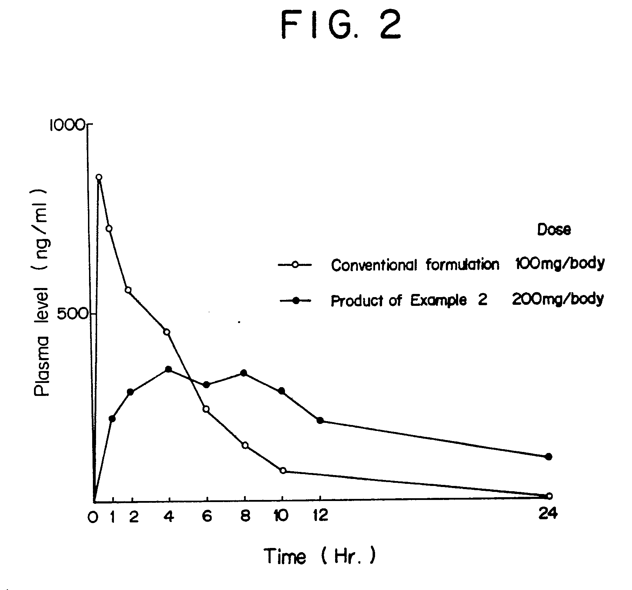

By the method of Example 1, but using beagle dogs, the amosulalol hydrochloride concentration in the plasma was measured after administration of the formulation of this invention and of the conventional formulation. The results are shown in Fig. 2.

As shown in Fig. 2, it can be seen that in the case of using the formulation of this invention, a suitable level of amosulalol hydrochloride in the plasma is maintained better than when using the conventional formulation.

Example 3

In a vertical mixer were mixed 200 g of amosulalol hydrochloride, 30 g of citric acid and 60 g of hydroxypropylmethyl cellulose phthalate (HP-55, trade name) and after adding gradually thereto 64 g of an aqueous solution of 10% hydroxypropyl cellulose under stirring, granules were formed from the mixture. After drying granules thus formed for 4 hours at 40°C, 1.6 g of magnesium stearate was added to the granules and the resultant mixture was formed into tablets by means of a tableting machine.

By the method of Example 2, the amosulalol hydrochloride concentration in the plasma was measured following use of the formulation of this invention and of aqueous 1% amosulalol hydrochloride.

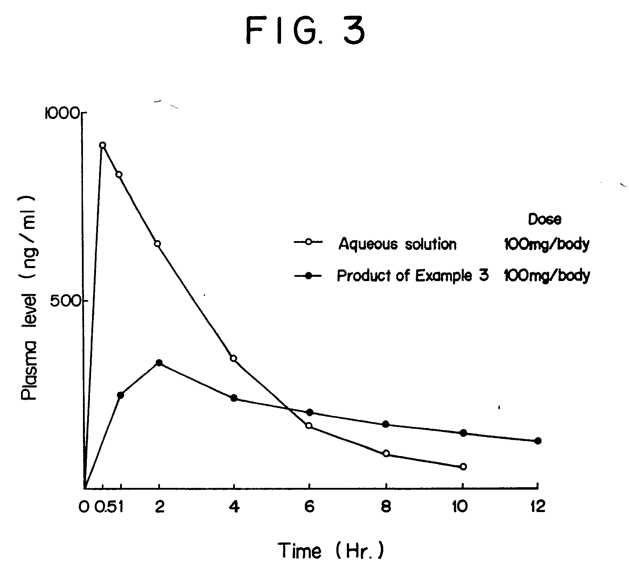

The results are shown in Fig. 3.

As shown in Fig. 3, it can be seen that in the case of using the formulation of this invention, a suitable level of amosulalol in the plasma was maintained better than when using the aqueous solution of amosulalol hydrochloride.

**Claims for the Contracting States BE, CH, DE, FR, GB, IT, LI, NL, SE**

1. An amosulalol hydrochloride formulation comprising amosulalol hydrochloride characterised in that the formulation further comprises between 10% and 30% by weight of an entero-soluble material in intimate admixture.

2. An amosulalol hydrochloride formulation according to claim 1 further comprising a pharmaceutically acceptable organic acid.

3. A formulation as claimed in claim 2, wherein the organic acid is selected from citric and tartaric acids.

4. A formulation as claimed in claim 1, 2 or 3 wherein the entero-soluble material comprises methacrylic acid/ethyl acrylate copolymer.

5. A method for producing an amosulalol formulation, characterised by intimately admixing amosulalol hydrochloride and between 10% and 30% by weight of an entero-soluble material.

**Claims for the Contracting State: AT**

1. A process for producing a long acting formulation of amosulalol hydrochloride which comprises adding 10-30% w/w entero-soluble material or a solution or a suspension thereof in water or organic solvent, to amosulalol hydrochloride or mixture of amosulalol hydrochloride and excipient, granulating the resultant intimate admixture if desired by using binder, and tableting the resultant granules by tableting machine.

2. The process as claimed in claim 1, wherein the entero-soluble material is methacrylic acid/ethylacrylate copolymer.

3. A process of producing a long acting formulation of amosulalol hydrochloride which comprises adding 10-30% w/w entero-soluble material, or a solution or a suspension thereof in water or organic solvent, to amosulalol hydrochloride or mixture of amosulalol hydrochloride and excipient, adding pharmaceutically acceptable organic acid or a solution thereof to amosulalol hydrochloride or mixture of amosulalol hydrochloride and excipient before or after the addition of entero-soluble material, granulating the resultant intimate admixture, if desired by using binder, and tableting the resultant granules by tableting machine.

4. The process as claimed in claim 3, wherein the entero-soluble material is methacrylic acid/ethylacrylate copolymer.

5. The process as claimed in claim 3, wherein the pharmaceutically acceptable organic acid is citric acid or tartaric acid.

**Patentansprüche für die Vertragsstaaten: BE, CH, DE, FR, GB, IT, LI, NL, SE**

1. Amosulalolhydrochlorid-Formulierung, enthaltend Amosulalolhydrochlorid, dadurch gekennzeichnet, daß die Formulierung weiterhin in innigem Gemisch 10 bis 30 Gew.-% eines darmlöslichen Materials enthält.

2. Amosulalolhydrochlorid-Formulierung nach Anspruch 1, dadurch gekennzeichnet, daß sie zusätzlich eine pharmazeutisch annehmbare organische Säure enthält.

3. Formulierung nach Anspruch 2, dadurch gekennzeichnet, daß die organische Säure aus Zitronensäure und Weinsäure ausgewählt ist.

4. Formulierung nach Anspruch 1, 2 oder 3, dadurch gekennzeichnet, daß das darmlösliche Material (ein) Methacrylsäure/Ethylacrylat-Copolymer umfaßt.

5. Verfahren zur Herstellung einer Amosulalol-Formulierung, dadurch gekennzeichnet, daß man Amosulalolhydrochlorid und 10 bis 30 Gew.-% eines darmlöslichen Materials innig miteinander vermischt.

**Patentansprüche für den Vertragsstaat: AT**

1. Verfahren zur Herstellung einer Amosulalolhydrochlorid-Formulierung mit langanhaltender Wirkung, dadurch gekennzeichnet, daß man 10 bis 30% Gew./Gew. eines darmlöslichen Materials oder einer Lösung oder einer Suspension davon in Wasser oder einem organischen Lösungsmittel zu Amosulalolhydrochlorid oder einem Gemisch aus Amosulalolhydrochlorid und Exzipiens gibt, das erhaltene innige Gemisch Granuliert, gegebenenfalls unter Verwendung eines Bindemittels, und die erhaltenen Granula mit einer Tablettiermaschine tablettiert.

2. Verfahren nach Anspruch 1, dadurch gekennzeichnet, daß das darmlösliche Material (ein) Methacrylsäure/Ethylacrylat-Copolymer ist.

3. Verfahren zur Herstellung einer Amosulalolhydrochlorid-Formulierung mit langanhaltender Wirkung, dadurch gekennzeichnet, daß man 10 bis 30% Gew./Gew. darmlösliches Material oder einer Lösung oder einer Suspension davon in Wasser oder organischem Lösungsmittel zu Amosulalolhydrochlorid oder einem Gemisch aus Amosulalolhydrochlorid oder Exzipiens gibt, eine pharmazeutisch annehmbare organische Säure oder eine Lösung davon zu Amosulalolhydrochlorid oder einem Gemisch aus Amosulalolhydrochlorid und Exzipiens vor oder nach der Zugabe des darmlöslichen Materials zusetzt, das entstandene innige Gemisch granuliert, gegebenenfalls unter Verwendung eines Bindemittels, und die erhaltenen Granula mit einer Tablettiermaschine tablettiert.

4. Verfahren nach Anspruch 3, dadurch gekennzeichnet, daß das darmlösliche Material (ein) Methacrylsäure/Ethylacrylat-Copolymer ist.

5. Verfahren nach Anspruch 3, dadurch gekennzeichnet, daß die pharmazeutisch annehmbare organische Säure Zitronensäure oder Weinsäure ist.

### Revendications pour les Etats Contractants: BE, CH, DE, FR, GB, IT, LI, NL, SE

1. Une formulation de chlorhydrate d'amosulalol comprenant du chlorhydrate d'amosulalol, caractérisée en ce que la formulation comprend en outre entre 10 et 30% en poids d'une matière entéro-soluble dans un mélange intime.

2. Une formulation de chlorhydrate d'amosulalol selon la revendication 1 comprenant en outre un acide organique pharmaceutiquement acceptable.

3. Une formulation comme revendiquée dans la revendication 2, dans laquelle l'acide organique est choisi parmi les acides citrique et tartrique.

4. Une formulation telle que revendiquée dans la revendication 1, 2 ou 3 dans laquelle la matière entéro-soluble comprend un copolymère acide méthacrylique/acrylate d'éthyle.

5. Un procédé pour fabriquer une formulation d'amosulalol caractérisé en ce qu'on mélange intimement du chlorhydrate d'amosulalol et entre 10 et 30% en poids d'une matière entéro-soluble.

### Revendications pour l'Etat Contractant: AT

1. Un procédé pour fabriquer une formulation à action prolongée de chlorhydrate d'amosulalol qui comprend l'addition de 10 à 30% en poids d'une matière entéro-soluble, ou d'une solution ou une suspension de celle-ci dans l'eau ou dans un solvant organique, à du chlorhydrate d'amosulalol ou à un mélange de chlorhydrate d'amosulalol et à un excipient, la granulation du mélange intime résultant si cela est désiré en utilisant un liant, et la mise sous comprimés des granulés résultants au moyen d'une machine à produire des comprimés.

2. Le procédé comme revendiqué dans la revendication 1, dans lequel la matière entéro-soluble est un copolymère acide méthacrylique/acrylate d'éthyle.

3. Un procédé pour fabriquer une formulation à action prolongée de chlorhydrate d'amosulalol qui comprend l'addition de 10-30% en poids d'une matière entéro-soluble, ou une solution ou une suspension de celle-ci dans l'eau ou dans un solvant organique, à du chlorhydrate d'amosulalol ou à un mélange de chlorhydrate d'amosulalol et à un excipient, l'addition d'un acide organique pharmaceutiquement acceptable ou d'une solution de celui-ci à du chlorhydrate d'amosulalol ou à un mélange de chlorhydrate d'amosulalol et d'un excipient avant ou après l'addition de la matière entéro-soluble, la granulation du mélange intime résultant si cela est désiré en utilisant un liant, et la mise sous comprimés des granulés résultants au moyen d'une machine à fabriquer des comprimés.

4. Le procédé comme revendiqué dans la revendication 3, dans lequel la matière entéro-soluble est un copolymère acide méthacrylique/acrylate d'ethyle.

5. Le procédé comme revendiqué dans la revendication 3, dans lequel l'acide organique pharmaceutiquement acceptable est de l'acide citrique ou de l'acide tartrique.

# F I G. 1

Plasma level ( ng / ml )

Dose

−o− Conventional formulation    25mg/body

−●− Product of Example 1     50mg/body

Time ( Hr. )

# FIG. 2

# FIG. 3